# EUROPEAN PATENT SPECIFICATION

(11) **EP 2 561 063 B1**
(45) Date of publication and mention of the grant of the patent: **04.01.2017**
(21) Application number: 11715569.7
(22) Date of filing: 22.04.2011
(51) Int. Cl.: C12N 1/20, C12N 1/38, A61K 39/102, C07K 14/285, C12R 1/21

(54) **A VACCINE COMPRISING INACTIVATED CELLS OF HAEMOPHILUS PARASUIS BACTERIA OF SEROTYPE 5**
HAEMOPHILUS PARASUIS-BAKTERIEN VOM SEROTYP 5 UND IMPFSTOFF MIT DIESEN BAKTERIEN
BACTÉRIE HAEMOPHILUS PARASUIS DE SÉROTYPE 5 ET VACCIN COMPRENANT CETTE BACTÉRIE

(30) Priority: 23.04.2010 EP 10160851; 23.04.2010 US 327340 P
(43) Date of publication of application: 27.02.2013
(73) Proprietor: Intervet International B.V., 5831 AN Boxmeer (NL)
(72) Inventor: WITVLIET, Maarten, Hendrik, NL-5831 AN Boxmeer (NL); HENSEN, Selma, Marianne, NL-5831 AN Boxmeer (NL); SEGERS, Ruud, Philip, Antoon, Maria, NL-5831 AN Boxmeer (NL)
(74) Representative: Intervet International B.V.
(86) International application number: PCT/EP2011/056495
(87) International publication number: WO 2011/131789

(56) References cited:
- EP-A1- 0 389 347
- WO-A1-95/21627
- WO-A1-2009/118273
- WO-A1-2009/118330
- WO-A2-00/01408
- WO-A2-2008/085406
- DEL RIO M L ET AL: "Identification and characterization of the TonB region and its role in transferrin-mediated iron acquisition in Haemophilus parasuis", FEMS IMMUNOLOGY AND MEDICAL MICROBIOLOGY, vol. 45, no. 1, 1 July 2005 (2005-07-01), pages 75-86, XP025316666, ISSN: 0928-8244 [retrieved on 2005-07-01]
- QIYUN XIE ET AL: "Transcriptional responses of Haemophilus parasuis to iron-restriction stress in vitro", BIOMETALS, vol. 22, no. 6, 30 April 2009 (2009-04-30) , pages 907-916, XP019746387, ISSN: 1572-8773
- MELNIKOW ET AL: "Microarray analysis of Haemophilus parasuis gene expression under in vitro growth conditions mimicking the in vivo environment", VETERINARY MICROBIOLOGY, vol. 110, no. 3-4, 31 October 2005 (2005-10-31), pages 255-263, XP005090234, ISSN: 0378-1135
- OLIVEIRA S ET AL: "Haemophilus parasuis: New trends on diagnosis, epidemiology and control", VETERINARY MICROBIOLOGY, vol. 99, no. 1, 26 March 2004 (2004-03-26) , pages 1-12, XP002484736, ISSN: 0378-1135
- RAPP-GABRIELSON V ET AL: "Haemophilus parasuis: immunity in swine after vaccination", VETERINARY MEDICINE, vol. January, 1 January 1997 (1997-01-01), pages 83-90, XP009105182, ISSN: 8750-7943 cited in the application
- BAK H ET AL: "Protection of vaccinated pigs against experimental infections with homologous and heterologous Haemophilus parasuis", VETERINARY RECORD, vol. 151, no. 17, 26 October 2002 (2002-10-26), pages 502-505, XP009101823, ISSN: 0042-4900
- OLIVEIRA S ET AL: "Evaluation of Haemophilus parasuis control in the nursery using vaccination and controlled exposure", JOURNAL OF SWINE HEALTH AND PRODUCTION, vol. 12, no. 3, 1 May 2004 (2004-05-01), pages 123-128, XP009105190, ISSN: 1537-209X cited in the application
- SOLANO-AGUILAR G I ET AL: "Protective role of maternal antibodies against Haemophilus parasuis infection", AMERICAN JOURNAL OF VETERINARY RESEARCH, vol. 60, no. 1, 1 January 1999 (1999-01-01), pages 81-87, XP009105166, ISSN: 0002-9645

## Description

The present invention pertains to a vaccine comprising inactivated cells of *Haemophilus parasuis* bacteria.

*Haemophilus parasuis* is an opportunistic pathogenic bacterium of the upper respiratory tract of pigs. It is the cause of Glässer's disease, a condition that results in a wide variety of clinical signs such as arthritis, pericarditis, pleuritis, polyserositis, meningitis and acute death. Glässer's disease is mainly a problem associated with the nursery of piglets. However, with the provision of new health technologies such as segregated early weaning (SEW) and the emergence of new pathogens such as porcine reproductive and respiratory syndrome (PRRS) virus, the incidence of Glässer's disease has increased. Firstly, *Haemophilus parasuis* problems are exacerbated by PRRS infection. Next to this, although SEW technologies have eliminated *Haemophilus parasuis* from certain herds, this has become a very serious problem for breeding stock companies: they aim to elevate health status of their animals and in the process increase the susceptibility of replacement stock to *Haemophilus parasuis.* Acute Glässer's disease is the frequent result when high-health-status replacement gilts or boars are introduced onto an infected farm. Therefore, among other things it has become of major importance to immunize naïve animals before introducing them onto infected farms. Generally speaking, obtaining protection against *Haemophilus parasuis* via immunization has become increasingly important. There are different serotypes known of *Haemophilus parasuis,* each of these can be identified using the technique of immunodiffusion (Kielstein et al. in J. Clin. Microbiol. 30:862-865; 1992 and Rapp-Gabrielson et al. in AJVR 53:659-664; 1992)

Combinord® is a registered inactivated vaccine (Intervet Denmark) to protect (i.e. to at least mitigate a negative effect of a disorder) sows and gilts against *Haemophilus parasuis* serotype 5. It also passively protects the offspring against serotype 5 via intake of the colostrum. However, it is not registered for protection against *Haemophilus parasuis* serotype 4. Indeed, as is generally known for inactivated vaccines that actively protect against *Haemophilus parasuis,* there is no cross protection of serotype 5 against serotype 4. This is known i.a. from a publication by Rapp-Gabrielson et al (Veterinary Medicine/January 1997/pp 83-90). Rapp-Gabrielson has examined differences in virulence and immunogenicity of strains of *Haemophilus parasuis,* focussing on protection and cross-protection against strains representing prevalent serotypes, viz. serotypes 5, 4, 13, 14, 2 and 12 respectively. Several conclusions could be drawn from this research. Most importantly, it was shown that *Haemophilus parasuis* serotype 5 provided no protection against serotype 4 challenge. Next to this, it was shown that within serotype 4, cross protection against heterologous strains was provided. Thus, protection against a strain of serotype 4 implied protection against other strains of the same serotype 4.

The above findings are in line with the findings by Oliveira (Journal of Swine Health and Production, Volume 12, Number 3, May and June 2004, pp 123-128. Here, it was established that with an inactivated *Haemophilus parasuis* vaccine, there is a lack of cross-protection between different serotypes. Fairly recently (March 21, 2006), Fort Dodge was granted marketing authorisation in the United Kingdom for an inactivated vaccine against *Haemophilus parasuis* serotypes 4 and 5 (Suvaxyn M.hyo - Parasuis). Indeed, the vaccine comprises both inactivated *Haemophilus parasuis* serotype 4 as well as *Haemophilus parasuis* serotype 5. The UK based RUMA (Responsible Use of Medicines in agriculture Alliance) has published guidelines on the use of vaccines and vaccination in pig production in November 2006. With respect to Glässer's disease these guidelines state that the commercial vaccines rely on strains from which there is little or no cross immunity to others.

It is noted that from WO 2008/085406 it is known that a composition comprising live modified *Haemophilus parasuis* bacteria of serotype 5, may provide protection against serotype 4. A disadvantage of a live composition when compared to an inactivated composition is the risk of side-effects due to replication of the bacteria in the subject animal.

The disadvantage of hitherto known *Haemophilus parasuis* bacteria of serotype 5 is that inactivated vaccines based solely thereon, can only provide active protection (via active immunisation) against bacteria of the same serotype. Thus, for active protection against the serotypes that are most prevalent, i.e. serotypes 4 and 5, both serotypes have to be in the inactivated vaccine. This is disadvantageous from a safety point of view (more bacteria present means a higher endotoxin content of the vaccine), but also from an economical point of view: the presence of two types of bacteria in the vaccine implies a higher chance of batch failure and a higher risk of contamination. Therefore, the chance of a batch being found unfit for sales is increased. It is therefore an object of the present invention to overcome or at least mitigate this disadvantage of the present *Haemophilus parasuis* bacteria of serotype 5.

To this end it has been found that *Haemophilus parasuis* bacteria of serotype 5 expressing one or more iron-restriction proteins (i.e proteins the expression of which is upregulated in wild-type bacteria when grown under iron-restriction conditions), may provide active cross-protection against *Haemophilus parasuis* bacteria of serotype 4. As is commonly known, a protein the expression of which is upregulated when a bacterium is grown under iron-restriction conditions, can be upregulated by providing actual iron-restrictions conditions (i.e. low or no iron available in the medium), but also by genetically modifying the bacterium such that the gene that is upregulated under iron-restriction conditions is upregulated independent of the amount of iron present in the cultivation medium. In any case, the present invention pertains to a novel *Haemophilus parasuis* serotype 5 bacterium that expresses at least one iron-restriction protein, which is the same protein the expression of which is upregulated when a wild-type *Haemophilus parasuis* serotype 5 bacterium is grown under iron-restriction conditions. Actual iron-restriction conditions are conditions in which the amount of available iron in the cultivation medium negatively influences the maximum obtainable growth rate of the bacterium in that medium (i.e. without any other changes of the constituents of the medium). That is, increasing the amount of iron present will lead to an increase of the growth rate of the bacterium in that medium. In contrast, under iron-replete conditions the growth rate of the bacterium is at a maximum. An increase in the amount of iron in the cultivation medium will thus not lead to an increase in the growth rate of the bacterium. It is noted that conditions of iron restriction can be provided in several art-known ways. One option is the addition of an agent to an iron-containing cultivating medium which agent chelates at least part of the iron present. Known iron-chelators are for example α,α-bipyridyl (also called "dipyridyl"), nitrilotriacetic acid, diethylenetriaminepentaacetic acid, desferrioxamine etc. This way, depending on the amount chelator used, one can obtain any iron concentration below a replete level. Other means are for example to pre-treat an iron-containing medium to remove iron by an ion exchanger, for example Chelex 100 (available from Biorad), or to obtain a defined low concentration of iron added to a (self-)assembled medium.

For *Haemophilus parasuis* bacteria of serotype 5, it was found that under iron-restriction conditions, the bacterium expresses several proteins (which may be free or bound proteins such as glycoproteins) at different rates when compared to cultivation under iron-replete conditions. At least one protein, which is very clearly visible around 60 kDa on a Western-blot when a wild-type bacterium is cultivated under iron-restriction conditions, is substantially less expressed, or even not expressed at all, when the bacterium is cultivated under iron-replete conditions. It is believed that the expression of such proteins induces the active protection against *Haemophilus parasuis* bacteria of other serotypes, in particular serotype 4. Given the prior art that specifically and persistently teaches that a vaccine containing inactivated cells of *Haemophilus parasuis* serotype 5 bacteria does not provide active protection against *Haemophilus parasuis* serotype 4 bacteria this could not have been reasonably expected. Note that many embodiments for obtaining a vaccine using the novel bacteria of the present invention can be devised. For example, the inactivated bacteria could be put in the vaccine as such, or could be used to obtain derivatives thereof for constituting a vaccine. In view of the latter mentioned derivatives, it is commonly known, in particular for outer membrane associated components, even more particularly for proteins specifically or increasingly expressed under iron-restriction conditions, that these sub-units may contribute in large to the effective immunological response of the target animal to the vaccine. As such, these and other derivatives of the original bacteria could be used in many cases as the sole components in the vaccine. As is commonly known, one can use various art-known methods to obtain such derivatives in (substantially) pure form, e.g. by having them made via a recombinant technique, by synthesizing the component or by purification of the component out of fermentation broth. One or more (preferably recombinant) subunits of the bacterium could be used as the actual antigens to formulate a vaccine. Such sub-units could be expressed in organisms other than *Haemophilus parasuis,* for example in *Escherichia coli, Salmonella enterica,* or Apicomplexan species, as a means for production or as a vector when immunizing an animal.

A vaccine in the sense of this invention is a constitution suitable for application to an animal, comprising one or more antigens such as attenuated or killed microorganisms and/or derivatives thereof in an immunologically effective amount (i.e. capable of stimulating the immune system of the target animal sufficiently to at least reduce the negative effects of a challenge with pathogenic micro-organisms), typically combined with a pharmaceutically acceptable carrier such as a liquid containing water, optionally comprising immunostimulating agents (adjuvants), which upon administration to the animal induces an immune response for treating a disease or disorder associated with a pathogenic (often wild-type) micro-organism, i.e. at least aids in preventing, ameliorating or curing the disease or disorder. This prevents, or at least diminishes, the level of infection or the clinical signs of the disease or disorder in the target animal, and consequently the severity of the disease or disorder. Also the further spread of the disease or disorder in the environment may be halted or diminished.

It is noted that from WO 2009/118330 it is known that offspring of sows or gilts immunized with *Haemophilus parasuis* bacteria of serotype 5, are passively protected against *Haemophilus parasuis* bacteria of serotype 4. However, there is no proof in this document that the vaccinated sows themselves are protected against serotype 4. On the contrary, it is known from the prior art (see the Rapp-Gabrielson article mentioned here-above) that this is not the case. Thus, it is not clear from the WO 2009/118330 reference that active protection against bacteria of serotype 4 can be induced in a target animal, by immunizing that animal with *Haemophilus parasuis* bacteria of serotype 5, grown under iron-restriction conditions. Given the fact that in the art, no one has ever explored or even suggested the route of cultivating *Haemophilus parasuis* serotype 5 bacteria under iron restriction conditions to induce cross-protection against bacteria of serotype 4, even though this route has been explored for other bacteria since many years (see i.a. EP 749 321 and EP 389 347), it is clear that this route has always been regarded as deemed unsuccessful for *Haemophilus parasuis* by vaccine specialists.

The vaccine comprises inactivated cells of the novel *Haemophilus parasuis* serotype 5 bacteria. It appears that a vaccine comprising inactivated cells provides sufficient protection. The advantage of an inactive antigen is safety. The *Haemophilus parasuis* serotype 5 bacteria may be inactivated by any art-known method, such as by using chemical inactivators such as beta-propiolactone, thimerosal or (another mercury donating agent), formaldehyde etc., applying physical methods such as heat, UV-light, micro-waves etc., by using biological methods such as enyzme-based methods to kill the bacteria, and any other method as is commonly applied in the art. It is noted that by using such methods, parts of the bacterial cells may lose their association with the cells. In particular, this might be the case with cell membrane associated components such as the outer membrane itself or outer membrane proteins.

In an embodiment, the vaccine comprises additional antigens corresponding to one or more micro-organisms in the group consisting of of *Actinobacillus pleuropneumoniae, Mycoplasma hyopneumoniae, Lawsonia intracellularis, Streptococcus suis, Salmonella serovars, Erysipelothrix rhusiopathiae,* porcine circo virus and PRRS (porcine reproductive and respiratory syndrome) virus. In a specific embodiment the vaccine comprises, next to antigens of *Haemophilus parasuis* bacteria according to the present invention, antigens of *Mycoplasma hyopneumoniae, Lawsonia intracellularis,* porcine circo virus and optionally *Erysipelothrix rhusiopathiae.* This vaccine could for example be obtained by mixing the antigens of *Haemophilus parasuis* bacteria, (immediately) prior to administration with a vaccine containing antigens of the other pathogens. In order to prevent that the ultimate combination vaccine comprises too much diluent, the antigens of the *Haemophilus parasuis* bacteria, preferably inactivated cells, should be present in freeze-dried form. This way, the mixing does not involve an increase in the amount of diluent.

Also described is a protein isolated from *Haemophilus parasuis* bacteria of serotype 5, grown under iron-restriction conditions, the protein being visible on a Western-blot when reacted with serum of a convalescent animal which has recovered from an infection by *Haemophilus parasuis* bacteria of serotype 4, and not being visible on a Western-blot when *Haemophilus parasuis* bacteria of serotype 5, grown under iron-replete conditions, are reacted under the same conditions. This protein can be used for example in a diagnostic test to allow discrimination between *Haemophilus* bacteria grown under iron-restriction conditions and *Haemophilus* bacteria grown under iron-replete conditions. Also, the protein may be useful as an antigen in a vaccine to protect against *Haemophilus parasuis* bacteria.

The invention will be explained by the following examples that pertain to a preferred embodiment of the present invention.
Figure 1 shows an analysis of *Haemophilus parasuis* serotype 5 vaccine antigen produced under iron-replete culture conditions (indicated as "+Fe") and under conditions of iron-restriction (indicated as "-Fe") by SDS-PAGE.
Figure 2 shows an analysis of *Haemophilus parasuis* serotype 5 vaccine antigen produced under iron-replete culture conditions (indicated as "+Fe") and under conditions of iron-restriction (indicated as "-Fe") by Western-blot.

### EXPERIMENTAL DESIGN

### Bacterial cultures

For the preparation of vaccine antigens, glycerol stocks of a *Haemophilus parasuis* serotype 4 and a *Haemophilus parasuis* serotype 5 strain are used. For each vaccine antigen batch 1 ampoule of glycerol stock is thawed and added to 100 ml RPMI (cultivation medium, commercially available from Invitrogen) + 10 g/l yeast extract + 0.4% v/v NAD in a 500 ml shake flask and cultured at 100 rpm, 37 °C for 16-24 hours. This is followed by subculturing in 125 ml of the same medium + 10 g/l yeast extract + 0.4 % v/v NAD in a 500 ml shake flask at a 10 % v/v inoculation rate and cultured at 100 rpm, 37 °C for 18-24 hours. For each vaccine antigen batch 2-4 shake flasks with 125 ml of culture each are made and pooled at the end of cultivation.

To induce iron-restriction conditions, 10 mM dipyridyl is added to a final concentration of 80µM when the culture has reached an optical density at 660 nm of 0.6. To inactivate the bacteria, formalin is added to a final concentration of 0.6 % v/v, followed by incubation for 2 hours at room temperature. Inactivated cultures are centrifuged for 10 minutes at 13,000 g. The supernatant is discarded and the bacterial cell pellets are resuspended in PBS at 1/6 of the original culture volume. The suspensions are used as vaccine antigens. To be able and formulate the final vaccines at equal vaccine antigen concentrations, the total nitrogen content of these suspensions is determined.

### Analysis of vaccine antigens for iron-restricted protein production

Vaccine antigens are analyzed by standard blotting techniques, SDS poly-acrylamide gel electrophoresis (SDS-PAGE), followed by Western blotting onto a PVDF membrane, in this case of a whole cell lysate (an outer membrane extract could for example also be used). A convalescent antiserum derived from a pig that has undergone a natural infection with *Haemophilus parasuis* serotype 4 is used to probe the PVDF membrane. More specifically, SDS-PAGE was performed using a NuPage 10% Bis-Tris gel (1.0 mm) under reducing conditions with a MOPS/MES SDS buffer, run on 200V/125mA during 64 minutes. To blot the gel onto an Immobilon P transfer membrane PVDF 0.45 um (Millipore), the semi-dry Western blotting method according to Towbin (Towbin, H; Staehlin, T. and Gordon, J., Proc. Nat. Acad. Sci. 76, 4350, 1979) was used. The blot was blocked with 100 ml 0.04 M PBS containing 0.5% Tween 20 (pH = 7.2) and 1% m/v milkpowder for one hour at 37°C. The blot was washed once with 0.04 M PBS and 0.5% Tween 20 (pH = 7.2) for 30 seconds. Subsequently, the blot was incubated for one hour at 37°C in 20 ml 0.04 M PBS containing 0.05% Tween 20 and 1% milkpowder containing a 125 times dilution of the pig serum, followed by washing three times for five minutes with 100 ml 0.04 M PBS containing 0.5% Tween 20 (pH = 7.2). Then the blot was incubated for one hour at 37°C with 20 ml 0.04 M PBS containing 0.05% Tween 20 and 1% milkpowder and 1000 times diluted Rabbit-anti-Swine (IgG)-HRP, followed by washing three times for five minutes with 100 ml 0.04 M PBS containing 0.5% Tween 20 (pH = 7.2). The blot was incubated in substrate Vector SG solution (Vector SG substrate kit for peroxidase (Vector, SK-4700)) until there was sufficient color development, i.e. a band visible with the naked human eye around 60 kDa for the iron-restriction sample. The reaction was stopped by washing for five minutes in distilled water.

### Vaccine formulation

The vaccine antigens to be tested in pigs *(Haemophilus parasuis* serotype 4 produced under iron-replete conditions and *Haemophilus parasuis* serotype 5 produced under iron-restriction conditions, the latter also being indicated as "IRP" bacteria) are diluted in PBS to a total nitrogen concentration of 0.05 µg/ml and mixed 1:1 with a dl-α-tocopheryl acetate-based adjuvant (Diluvac Forte, available from Intervet/Schering-Plough Animal Health). In general, any other vaccine comprising *H. parasuis* serotype 5 antigens produced under iron-restriction conditions can be formulated by using art-known methods that basically comprise admixing suitable antigens of *H. parasuis* (live or inactivated, whole cell, extract, purified fraction or subunit) with a pharmaceutically acceptable carrier, e.g. a liquid carrier such as (optionally buffered) water or a solid carrier such as commonly used to obtain freeze-dried vaccines. Optionally other substances such as adjuvants, stabilisers, viscosity modifiers or other components are added depending on the intended use or required properties of the vaccine.

### Vaccination

Three groups of 10 piglets from a *Haemophilus parasuis* free herd were used. Two vaccines (*Haemophilus parasuis* serotype 4 produced under iron-replete conditions and *Haemophilus parasuis* serotype 5 produced under iron-restriction conditions) were administered intramuscularly as a 2 ml dose at one and four weeks of age. The remaining 10 piglets were used as non-vaccinated negative control group. One of the piglets in the negative control group died before the challenge infection.

### Haemophilus parasuis serotype 4 challenge infection

At approximately seven weeks of age, all 29 piglets received a challenge infection. The challenge compound is a field isolate (live strain) of *Haemophilus parasuis* serotype 4. The isolate is obtained by using a method as known from the Oliveira reference as mentioned here-above. The challenge culture was freshly prepared prior to challenge. *Haemophilus parasuis* was cultured on chocolate agar plates with NAD at 37° for 24-72 hours. Bacteria were harvested with 2 to 5 ml CYS medium per plate and this was used to inoculate CYS medium with NAD (2 to 5 ml per 100 ml medium). This was followed by incubation at 37°C for ± 5 hours. Then, the culture was centrifuged for 10 min at 2,000 g and the pellet was resuspended in 0.04 M PBS. The challenge materials was stored on ice until use, which use was within 2 hours. The piglets were challenged with *Haemophilus parasuis* by the aerosol route in an aerosol box. This was done in groups of 10 piglets each. The aerosol was given by means of a Devilbis Nebulizer. The total amount of challenge culture per group of 10 piglets was 50 ml. The challenge culture contained ± 10⁸ cfu/ml. The piglets were left in the aerosol box for approximately 30 minutes.

After challenge, rectal temperature, general condition, locomotion, nervous system and other abnormalities were scored daily for a period of 10 days.

The scoring system for the general condition was as follows
0= normal
1= inappetant
2= inappetant and depressed
3= depressed and slow to rise
4= moribund.

The scoring system for the locomotion was as follows:
0= normal behaviour
1 = swollen, hot or painful joints
2= limping on one or more legs
3= failure to put a foot to the ground
4= refusal to stand if not moribund.

The scoring system for the nervous system was as follows:
0= normal behavior
1 = tilted head
2= loss of balance
4= convulsions

The scoring system for other abnormalities was as follows:
0= no abnormalities
2= minor abnormalities
4= severe abnormality requiring euthanasia.

Any piglet that was euthanized was automatically given a score of 4 for the category of clinical sign that led to the humane endpoint decision. The total clinical score per piglet was the sum of daily clinical scores from challenge to post mortem examination.

Necropsy was done on all animals that died after challenge, and the surviving animals were necropsied on day 10 post challenge.
At post-mortem examination, gross pathological lesions of the peritoneal, pleural and pericardial cavity and affected joints were scored as follows:
0 = no abnormalities detected.
1 = minimal fibrin deposits and/or minimal amount of clear fluid
2 = mild fibrinous serositis and/or mild accumulation of clear fluid/increase in synovia
3 = moderate fibrinous serositis and/or moderate accumulation of clear fluid/increase in synovia or mild signs of purulent exudation.
4 = severe fibrinous serositis and/or severe accumulation of clear fluid/increase in synovia or moderate signs of purulent exudation.

The total post-mortem score per piglet is the sum of the individual score for each cavity (abdominal, pleural and pericardial) and the affected joints.

### RESULTS

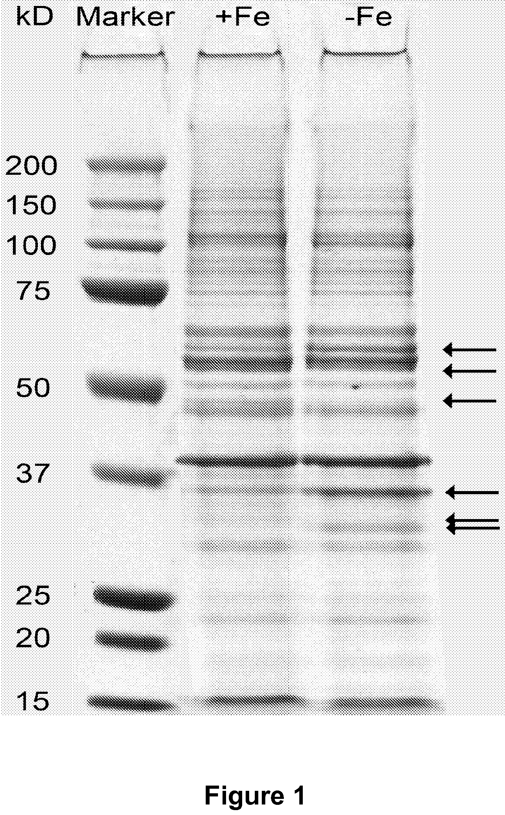

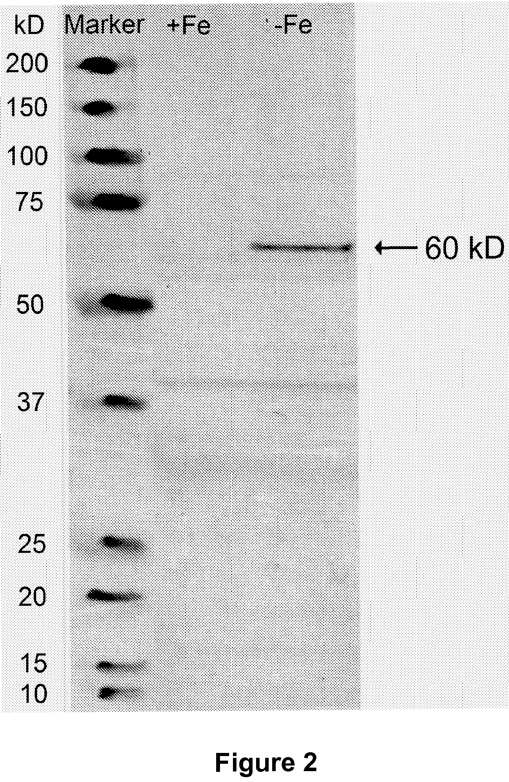

Figure 1 shows an analysis of *Haemophilus parasuis* serotype 5 vaccine antigen produced under iron-replete culture conditions (indicated as "**+Fe**") and under conditions of iron-restriction (indicated as "**-Fe**") by SDS-PAGE. Figure 2 shows a corresponding analysis by additional Western blotting. By SDS-PAGE and Western-blotting differences in protein expression are observed (indicated by the arrows). Most notably is the reaction of antibodies in the convalescent pig serum to a protein band with an apparent molecular weight of approximately 60 kDa in the vaccine antigen cultured under conditions of iron-restriction, blotted as indicated here-above. Under these blotting conditions, no band is visible in the vaccine antigen cultured under iron-replete conditions at 60 kDa (it may however be that a vague band will ultimately become visible when colour development is not stopped as soon as a clear band is visible for the iron-restriction sample). This 60 kDa protein is not observed when *Haemophilus parasuis* serotype 4 is cultured on standard conditions (data not shown).

Table 1 shows the effects of the *Haemophilus parasuis* serotype 4 challenge infection on the two vaccinated groups and on the unvaccinated controls. Significant reductions of clinical signs, mortality and post mortem scores in the animals actively immunized with the *Haemophilus parasuis* type 4 vaccine or the *Haemophilus parasuis* type 5 IRP vaccine compared to the control piglets were observed. Mean temperature after challenge (data not shown) was comparable for both vaccines and significantly lower than controls. Although the serotype 5 IRP vaccine seemed to provide even better protection than the serotype 4 vaccine against a serotype 4 challange, the difference between the two vaccine groups was not statistically significant. Still, it can be concluded that by using *Haemophilus parasuis* serotype 5 IRP bacteria for constituting a vaccine for pigs, one can protect them at least to the same level against an infection with *Haemophilus parasuis* serotype 4 as compared to a situation wherein a *Haemophilus parasuis* serotype 4 vaccine is used.

**Table 1 Results for protection of the piglets**

| Vaccine | No of piglets | Mean clinical score | Mortality [n/ntot] | Mean post-mortem score |
|---|---|---|---|---|
| *H. parasuis* serotype 4 | 10 | 14.2^{*} | 2/10^{*} | 2.8^{*} |
| *H. parasuis* serotype 5 IRP | 10 | 6.5^{*} | 1 /10^{*} | 1.9^{*} |
| None | 9 | 52.2 | 7/9 | 7.0 |

| | | | | |
|---|---|---|---|---|
| *: significantly different from controls (p<0.05, Mann-Whitney U-test for scores and Fischer exact-test for mortality rate) | | | | |

## Claims

1. A vaccine comprising inactivated cells of *Haemophilus parasuis* bacteria of serotype 5 obtained by growing under iron-restrictions conditions, or derivates thereof, which bacteria express a protein whose expression is upregulated when the expressing bacterium is grown under iron-restriction conditions, visible on a Western-blot when reacted with serum of a convalescent animal which has recovered from an infection by *Haemophilus parasuis* bacteria of serotype 4, the said protein not being visible on a Western-blot when wild-type *Haemophilus parasuis* bacteria of serotype 5 grown under iron-replete conditions are reacted under the same conditions, the vaccine providing protection against a disorder arising from *Haemophilus parasuis* serotype 4 bacteria.

2. A vaccine according claim 1, **characterised in that** the vaccine comprises additional antigens corresponding to one or more micro-organisms in the group consisting of *Actinobacillus pleuropneumoniae, Mycoplasma hyopneumoniae, Lawsonia intracellularis, Streptococcus suis, Salmonella serovars, Erysipelothrix rhusiopathiae,* porcine circo virus, PRRS virus.

3. A vaccine according to claim 2, characterised that the vaccine comprises additional antigens of *Mycoplasma hyopneumoniae, Lawsonia intracellularis,* porcine circo virus and optionally *Erysipelothrix rhusiopathiae.*

4. A vaccine according to claim 3, **characterised in that** the vaccine is obtained by mixing inactivated cells of the *Haemophilus parasuis* bacteria, optionally in freeze-dried form, with a vaccine containing antigens of *Mycoplasma hyopneumoniae, Lawsonia intracellularis* and porcine circo virus.

## Patentansprüche

1. Impfstoff, der inaktivierte Zellen von *Haemophilus parasuis-*Bakterien des Serotyps 5, die durch Züchten unter Eisenbegrenzungsbedingungen erlangt wurden, oder Derivate davon umfasst, wobei die Bakterien ein Protein exprimieren, dessen Expression hochreguliert ist, wenn das exprimierende Bakterium unter Eisenbegrenzungsbedingungen gezüchtet wird, was auf einem Western Blot sichtbar ist, wenn sie mit dem Serum eines genesenden Tieres reagiert werden, das sich von einer Infektion durch *Haemophilus parasuis*-Bakterien des Serotyps 4 erholt hat, wobei das Protein auf einem Western Blot nicht sichtbar ist, wenn Wildtyp-*Raemophilus parasuis*-Bakterien des Serotyps 5, die unter Bedingungen mit reichlich Eisen gezüchtet wurden, unter den gleichen Bedingungen reagiert werden, wobei der Impfstoff Schutz gegen eine Erkrankung bietet, die sich aus *Haemophilus parasuis-*Bakterien Serotyp 4 entwickelt.

2. Impfstoff nach Anspruch 1, **dadurch gekennzeichnet, dass** der Impfstoff zusätzliche Antigene umfasst, die einem oder mehreren Mikroorganismen in der Gruppe bestehend aus *Actinobacillus pleuropneumoniae, Mykoplasma hyopneumoniae, Lawsonia intracellularis, Streptokokkus suis, Salmonella serovars, Erysipelothrix rhusiopathiae,* Porcine Circovirus, PRRS-Virus entsprechen.

3. Impfstoff nach Anspruch 2, **dadurch gekennzeichnet, dass** der Impfstoff zusätzliche Antigene von *Mykoplasma hyopneumoniae, Lawsonia intracellularis,* Porcine Circovirus und optional *Erysipelothrix rhusiopathiae umfasst.*

4. Impfstoff nach Anspruch 3, **dadurch gekennzeichnet, dass** der Impfstoff durch Mischen inaktivierter Zellen von den *Haemophilus parasuis*-Bakterien, optional in gefriergetrockneter Form, mit einem Impfstoff, der Antigene von *Mykoplasma hyopneumoniae, Lawsonia intracellularis* und Porcine Circovirus enthält, erlangt wird.

## Revendications

1. Vaccin comprenant des cellules inactivées de *bactéries Haemophilus parasuis* de sérotype 5 obtenues par culture dans des conditions avec une teneur en fer restreinte, ou leurs dérivés, lesquelles bactéries expriment une protéine dont l'expression est régulée à la hausse lorsque la bactérie d'expression est cultivée dans des conditions avec une teneur en fer restreinte, visible dans une analyse, western blot, lors de la mise en réaction avec le sérum d'un animal convalescent qui s'est remis d'une infection par *des bactéries Haemophilus parasuis* de sérotype 4, ladite protéine n'étant pas visible dans une analyse western blot lorsque des *bactéries Haemophilus parasuis* de sérotype 5 de type sauvage cultivées dans des conditions avec une teneur en fer restreinte sont mises en réaction dans les mêmes conditions, le vaccin fournissant une protection contre un trouble résultant de *bactéries Haemophilus parasuis* de sérotype 4.

2. Vaccin selon la revendication 1, **caractérisé en ce que** le vaccin comprend des antigènes supplémentaires correspondant à un ou plusieurs microorganismes du groupe consistant en *Actinobacillus pleuropneumoniae, Mycoplasma hyopneumoniae, Lawsonia intracellularis, Streptococcus suis, Salmonella serovars, Erysipelothrix rhusiopathiae,* Circovirus porcin, virus du syndrome dysgénésique et respiratoire du porc, PRRS.

3. Vaccin selon la revendication 2, **caractérisé en ce que** le vaccin comprend des antigènes supplémentaires de *Mycoplasma hyopneumoniae, de Lawsonia intracellularis,* de Circovirus porcin et éventuellement *d'Erysipelothrix rhusiopathiae.*

4. Vaccin selon la revendication 3, **caractérisé en ce que** le vaccin est obtenu en mélangeant des cellules inactivées de *bactéries Haemophilus parasuis,* éventuellement sous forme lyophilisée, avec un vaccin contenant des antigènes de *Mycoplasma hyopneumoniae, de Lawsonia intracellularis,* et de Circovirus porcin.
